# EUROPEAN PATENT APPLICATION

(11) **EP 2 722 036 A1**
(43) Date of publication of application: **23.04.2014**
(21) Application number: 13189184.8
(22) Date of filing: 17.10.2013
(51) Int. Cl.: A61K 9/16, A61K 9/20, A61K 31/4365, A61K 9/28

(54) **Solid oral formulations of prasugrel**

(30) Priority: 19.10.2012 TR 201212084; 30.09.2013 TR 201311421
(71) Applicant: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Saydam, Mehtap, 34460 Istanbul (TR)
(74) Representative: Sevinç, Erkan

(57) **Abstract**

The present invention relates to solid oral pharmaceutical formulations comprising prasugrel free base and one or more pharmaceutically acceptable excipient and process for preparing such a formulation.

## Description

### Field of Invention

The present invention relates to solid oral pharmaceutical formulations comprising prasugrel free base and one or more pharmaceutically acceptable excipient and process for preparing such a formulation.

### Background of Invention

Prasugrel hydrochloride is a member of the thienopyridine class and inhibits the activation and aggregation of platelets by means of P2Y12 and ADP receptors. Its chemical name is 2-acetoxy-5-(α-cyclopropylcarbonyl-2-fluorobenzyl)-4,5,6,7-tetrahydrothieno[3,2-c]pyridine hydrochloride, with the chemical structure illustrated below in Formula 1.

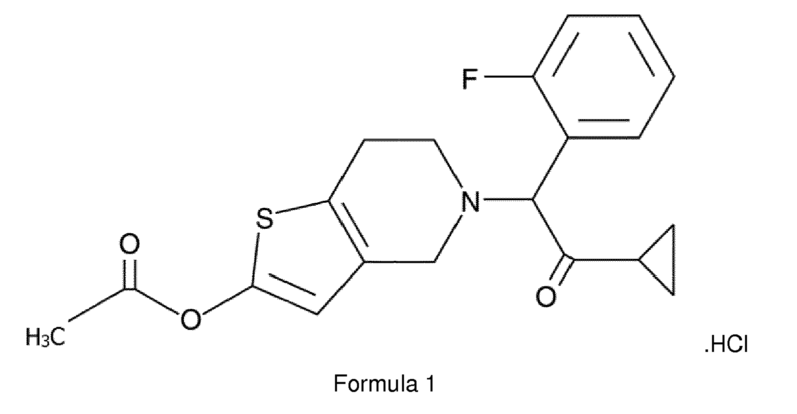

Prasugrel hydrochloride is a white and solid. It is well soluble at pH 2, weakly soluble at pH 3 to 4, and is substantially insoluble at pH 6 to 7.5.

Prasugrel hydrochloride is marketed under the trademark Effient^{®} in 5 or 10 mg dosages. It is initially administered in a dosage amount of 60 mg as a loading dose. It is used in preventing or treating thrombosis and cardiovascular diseases.

Searching the patent literature reveals various patents in relation to prasugrel.

The patent application EP1298132 discloses hydrochloride salt of prasugrel, a method for preparing this salt, and its use for thrombosis and embolism.

The patent application EP1728794 discloses maleate salt prasugrel, a method for preparing this salt, and its use for thrombosis and embolism.

The patent application WO2006135605 discloses a formulation containing prasugrel hydrochloride, packaged in a blister package which is air- and moisture-impermeable, and is inert against gas.

Patent application WO2008073759 discloses a tablet or capsule formulation containing prasugrel, packaged in an air- and moisture-impermeable blister under a gas with positive fluidity pressure in order to reduce the amount of oxygen and humidity generated during packaging.

Stability-related problems occur in many active agents, including prasugrel, under the influence of environmental and physical conditions. However, prasugrel is an active agent that is highly-susceptible to air and humidity. When prasugrel is exposed to air and humidity, it degrades structurally and develops chemical behavioral changes. As a result of this, two main problems emerge. The first problem is that the stability of the products developed is not at a desired level and the shelf life thereof is shortened. The second problem is that prasugrel reacts with the excipients employed in developing formulations containing prasugrel. This fact causes impurities to occur in the formulation and leads to incorporation of undesired components into the formulation. In addition to these, measures should be taken while preparing, packaging, and storing finished dosage forms comprising prasugrel.

As a consequence, a need rises for formulations of prasugrel and a process for preparing such formulations that overcome the problems mentioned above.

The present invention offers a prasugrel formulation that is resistant against environmental and physical conditions yielding better stability and longer shelf life. Also, this invention prevents the impurity formation in the formulation and presents improved dissolution profile. Moreover, the formulation is stable under production, packaging and storage conditions.

A further object of the present invention is to provide a simple, cost-effective, and time-saving process for preparing a prasugrel formulation.

Advantages and embodiments of the present invention will become apparent from the following description.

### Detailed Description of Invention

The objects of the present invention is to provide an improved prasugrel formulation which overcomes above described problems such as stability, short shelf life, impurity formation in the formulation and drawbacks in preparing, packaging and storing, and also to improve dissolution profile with using prasugrel free base and appropriate excipients in specific amounts.

In pharmaceutical formulations comprising prasugrel as an active agent, the salt of prasugrel, particularly the hydrochloride or maleate salt, is used. For instance, in the commercially available product Effient®, the active agent is prasugrel hydrochloride. However, the pharmaceutical formulation according to the present invention comprises prasugrel free base as an active agent. In other words, the formulation according to the present invention is substantially free of other forms of prasugrel, except for the free base of prasugrel. Using prasugrel free base which purified from the other forms of prasugrel as the active agent brings the stability of the formulation to a desired level. Accordingly, in this invention, it has surprisingly found that pharmaceutical formulations comprising prasugrel free base is more resistant against environmental and physical conditions as compared to pharmaceutical formulations comprising other forms of prasugrel that are particularly the hydrochloride and maleate salts of prasugrel.

In this embodiment of the present invention, the amount of prasugrel free base is from 1 to 80%, preferably from 3.5 to 60%, of the total weight of the tablet.

The pharmaceutical formulations according to the present invention may also comprise one or more pharmaceutically acceptable excipient(s). Pharmaceutically acceptable excipients comprise, but are not limited to, fillers or diluents, disintegrants, binders, lubricants, glidants, sweeteners, aromatic agents, preservatives, coloring agents and the like, and the mixtures thereof. Excipients widely used in dosage forms and the examples thereof are given below.

In another embodiment of the present invention, said fillers or diluents comprise, but are not limited to dibasic calcium phosphate, pullulan, maltodextrin, isomalt, sugar pellets, mannitol, spray-dried mannitol, microcrystalline cellulose, dibasic calcium phosphate dihydrate, lactose, sugars, sorbitol, mixture of microcrystalline cellulose and guar gum (Avicel CE-15), mixture of mannitol, polyplasdone and syloid (Pharmaburst), mixture of mannitol, crospovidone and polyvinyl acetate (Ludiflash), isomalt, Panexcea, F-Melt, sucrose, calcium salts and similar inorganic salts, heavy magnesium carbonate and the like, and the mixtures thereof. Preferably, it is dibasic calcium phosphate or pullulan.

In another embodiment of the present invention, said disintegrants comprise, but are not limited to, at least one or a mixture of sodium starch glycolate, croscarmellose sodium, crospovidone, sodium alginate, gums, starch, and magnesium aluminum silicate.

In a preferred embodiment of the present invention, said binders comprise, but are not limited to, low-substituted hydroxypropyl cellulose, xanthan gum, polyvinylpyrrolidone (povidone), gelatin, sugars, glucose, natural gums, gums, synthetic celluloses, polymethacrylate, hydroxypropyl methylcellulose, hydroxypropyl cellulose, carboxymethyl cellulose, methyl cellulose, and other cellulose derivatives and the like, and the mixtures thereof. Preferably, it is low-substituted hydroxypropyl cellulose.

In a preferred embodiment of the present invention, low substituted hydroxypropyl cellulose wherein its viscosity is between 6.0-10.0 mPa.s. is used as a binder.

The viscosity of the low substituted hydroxypropyl cellulose is measured by the method described in European Pharmacopoeia 7.0 Hydroxypropyl cellulose monograph. The said method is given as following:
While stirring, introduce a quantity of the substance to be examined equivalent to 6.00 g of the dried substance into 150 g of water R heated to 90 °C. Stir with a propeller-type stirrer for 10 min, place the flask in a bath of iced water, continue the stirring and
allow to remain in the bath of iced water for 40 min to ensure that dissolution is complete. Adjust the mass of the solution to 300 g and centrifuge the solution to expel any entrapped air. Adjust the temperature of the solution to 20 ± 0.1 °C. Determine the viscosity with a rotating viscometer at 20 °C and shear rate of 10 s⁻¹.

In this pharmaceutical composition of the present invention, it has surprisingly found that using low-substituted hydroxypropyl cellulose wherein its specific weight percentage is between 1% and 50%, preferably 6% and 25% based on the total weight of the tablet, provides desirable dissolution profile.

The dissolution method of prasugrel free base tablet is USP Apparatus II Paddle 75 rpm 900 ml Medium: Citrate-Phosphate buffer (0.023 M Citric acid + 0.026 M Sodium Phosphate, Dibasic), pH 4.

In a preferred embodiment of the present invention, said lubricants comprise, but are not limited to sodium stearyl fumarate, sodium lauryl sulphate, magnesium stearate, polyethylene glycol, metal stearates, hydrogenated castor oil and the like, and the mixtures thereof. Preferably, it is sodium stearyl fumarate.

It is known that magnesium stearate has some disadvantages despite being a good lubricant and because of this it is used in small quantities during drug manufacturing process. Magnesium stearate is practically insoluble in water and because of this hydrophobic characteristic it may retard the dissolution of a drug from a solid dosage form such as tablet or capsule. Tablet and especially capsule dissolution is sensitive to both the amount of magnesium stearate in the formulation and the blending time. Blending time should be limited. Long blending times can result in the formulation of hydrophobic powder beds that do not disperse easily and overblending can cause compaction problems. Tablet dissolution rate and crushing strength decreased as the time of blending increased; and magnesium stearate may also increase tablet friability. Blending times with magnesium stearate should therefore be carefully controlled. (Handbook of Pharmaceutical Excipients, fifth edition, Rowe, Raymond C., Sheskey, Paul J., Owen, Sian C., pages 430-432*).*

Sodium stearyl fumarate is extremely effective lubricant and less hydrophobic than magnesium stearate and has a less retardant effect on tablet dissolution than magnesium stearate. Sodium stearyl fumarate also doesn't have the over blending problems seen with magnesium steare. (Handbook of Pharmaceutical Excipients, fifth edition, Rowe, Raymond C., Sheskey, Paul J., Owen, Sian C., pages 705-707*).*

In this pharmaceutical composition of the present invention, it is objected to overcome the technical problems which are shown above by using sodium stearyl fumarate instead of magnesium stearate as a lubricant wherein the specific percentage amount of sodium stearyl fumarate in a formulation according to the present invention is between 0.1% and 10%, preferably 0.25% and 2% based on the total weight of the tablet. It has surprisingly found that the specific weight percentage of sodium stearyl fumarate leads to increase the manufacturing process quality in solid dosage forms.

In a preferred embodiment of the present invention, said glidants comprise, but are not limited to, stearic acid, colloidal silicon dioxide, talk, aluminium silicate and the like, and the mixtures thereof. Preferably, it is stearic acid.

In a preferred embodiment of the present invention, said sweeteners comprise, but are not limited to, aspartame, sucralose, saccharine; glucose, lactose, fructose and other sugars, and mannitol, sorbitol, xylitol, erythritol and other sugar alcohols and the like, and the mixtures thereof.

In a preferred embodiment of the present invention, said aromatic agents comprise, but are not limited to, menthol, mint, cinnamon, chocolate, vanillin, and fruit extracts such as of cherry, orange, strawberry, grape and the like, and the mixtures thereof.

The following additional excipients can also be used in the pharmaceutical formulation according to the present invention:
Coating agents, including, but not limited to, polymers such as hydroxypropyl methylcellulose, polyethylene glycol, polyvinylpyrrolidone, polyvinylpyrrolidone-vinyl acetate copolymer (PVP-VA), pullulan, all kinds of Opadry, Kollicoat IR as well as pigments, dyes, titanium dioxide and iron oxide, talk.

Colorants, including, but not limited to, Food, Drug, and Cosmetic (FD&C) dyes (e.g. FD&C blue, FD&C green, FD&C red, FD&C yellow, FD&C lake), ponceau, indigo Drug & Cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow and the like, and the mixtures thereof.

Preservatives, including, but not limited to, methylparaben and propylparaben and the salts thereof (e.g. sodium or potassium salts), sodium benzoate, citric acid, benzoic acid, butylated hydroxytoluene and butylated hydroxyanisole and the like, and the mixtures thereof.

The formulation according to the present invention may be in the form of a tablet, dragee, capsule, caplet, orally-disintegrating tablet, film-coated tablet, enteric tablet, buccal tablet, sublingual tablet, chewable tablet, effervescent tablet, rapid-release tablet, sachet, granule, pilule, pellet and similar solid oral dosage forms. The preferred dosage form according to the present invention is the solid dosage form, preferably a tablet form.

The present invention is used for treating thrombosis and cardiovascular diseases. These formulations can be used in the content of medicaments which are efficient in preventing or treating thrombosis and cardiovascular diseases.

The pharmaceutical formulation according to the present invention comprising prasugrel free base as an active agent offers improved stability therefore the formulation is unexpectedly resistant against environmental and physical conditions and eliminate the stability problems for production, packaging and storage conditions. In addition, dissolution profile is improved. Moreover, the present invention provides a simple, cost-effective, and time-saving process for preparing a prasugrel formulation.

This invention is further defined by reference to the following examples. Although the examples are not intended to limit the scope of the present invention, it should be considered in the light of the description detailed above.

### Example 1

| **Formulation Content** | **Amount (%)** |
|---|---|
| Prasugrel free base | 3.5-60 |
| Dibasic calcium phosphate | 5-90 |
| Low-substituted hydroxypropyl cellulose (L-HPC) | 6-25 |
| Pulluan | 0.1-25 |
| Stearic acid | 0.1-0.2 |
| Sodium stearyl fumarate | 0.25-2.0 |

The pharmaceutical formulation mentioned above is prepared as following: Prasugrel free base, L-HPC and dibasic calcium phosphate are mixed and made granules with alcoholic/ hydroalcoholic pullulan solution and obtained granules are dried and sieved. Then, the dried and sieved granules are first mixed with strearic acid and further with sodium stearyl fumarate and compressed as tablets. The obtained tablets may be coated with Opadry AMB/ Collicoat IR based on polyvinyl alcohol.

### Example 2

| **Formulation Content** | **Amount (%)** |
|---|---|
| Prasugrel free base | 3.5-60 |
| Maltodextrin | 5-90 |
| Low-substituted hydroxypropyl cellulose (L-HPC) | 6-25 |
| Sodium lauryl sulfate | 0.1-0.2 |
| Sodium stearyl fumarate | 0.25-2.0 |

The pharmaceutical formulation mentioned above is prepared as following: Prasugrel free base, L-HPC and maltodextrin are mixed. Then, the mixture is first mixed with sodium lauryl sulfate and then mixed with sodium stearyl fumarate and compressed as tablets. The obtained tablets may be coated with Opadry AMB/ Collicoat IR based on polyvinyl alcohol.

### Example 3

| **Formulation Content** | **Amount (%)** |
|---|---|
| Prasugrel free base | 3.5-60 |
| Isomalt | 5-90 |
| Xanthan gum | 0.05-20 |
| Low-substituted hydroxypropyl cellulose (L-HPC) | 6-25 |
| Sodium lauryl sulfate | 0.1-0.2 |
| Sodium stearyl fumarate | 0.25-2.0 |

The pharmaceutical formulation mentioned above is prepared as following: Prasugrel free base, isomalt, xanthan gum, L-HPC and sodium lauryl sulfate are mixed. Then, the mixture is mixed with a portion of sodium stearyl fumarate for short time and to be passed from roller compactor. The obtained granules are sieved and then mixed with the remaining sodium stearyl fumarate for short time and compressed as tablets or maybe filled to capsules. The obtained tablets may be coated with Opadry AMB/ Collicoat IR based on polyvinyl alcohol.

### Example 4

| **Formulation Content** | **Amount (%)** |
|---|---|
| Prasugrel free base | 3.5-60 |
| Pullulan | 0.1-25 |
| Sugar pellets | 5-90 |
| Low-substituted hydroxypropyl cellulose (L-HPC) | 6-25 |
| Sodium lauryl sulfate | 0.1-0.2 |
| Sodium stearyl fumarate | 0.25-2.0 |

The pharmaceutical formulation mentioned above is prepared as following: An alcoholic/ hydroalcoholic solution of Prasugrel free base with pullulan is prepared and the sugar pellets are coated with prepared solution. The obtained pellets are mixed with L-HPC and then first mixed with sodium lauryl sulfate and then mixed with sodium stearyl fumarate for short time and filled to capsules or compressed as tablets. The obtained tablets may be coated with Opadry AMB/ Kollicoat IR based on polyvinyl alcohol.

## Claims

1. A solid oral composition comprising prasugrel free base and pharmaceutically acceptable excipients.

2. The solid oral composition according to claim 1, **characterized in that** said composition is in tablet form.

3. The solid oral composition according to claims 1 and 2, **characterized in that** the amount of prasugrel free base is from 1 to 80%, preferably from 3.5 to 60% of the total weight of the tablet.

4. The solid oral composition according to claims 1 to 3, **characterized in that** the pharmaceutically acceptable excipients comprise fillers or diluents, disintegrants, binders, lubricants, glidants, sweeteners, aromatic agents, preservatives, coloring agents, and the mixtures thereof.

5. The solid oral composition according to claim 4, **characterized in that** said filler or diluent is selected from the group comprising dibasic calcium phosphate, pullulan, maltodextrin, isomalt, sugar pellets, mannitol, spray-dried mannitol, microcrystalline cellulose, dibasic calcium phosphate dihydrate, lactose, sugars, sorbitol, mixture of microcrystalline cellulose and guar gum (Avicel CE-15), mixture of mannitol, polyplasdone and syloid (Pharmaburst), mixture of mannitol, crospovidone and polyvinyl acetate (Ludiflash), isomalt, Panexcea, F-Melt, sucrose, calcium salts and similar inorganic salts, heavy magnesium carbonate and the like, and the mixtures thereof, preferably it is dibasic calcium phosphate or pullulan.

6. The solid oral composition according to claim 4, **characterized in that** said binder is selected from the group comprising low-substituted hydroxypropyl cellulose, xanthan gum, polyvinylpyrrolidone (povidone), gelatin, sugars, glucose, natural gums, gums, synthetic celluloses, polymethacrylate, hydroxypropyl methylcellulose, hydroxypropyl cellulose, carboxymethyl cellulose, methyl cellulose, and other cellulose derivatives and the like, and the mixtures thereof, preferably it is low-substituted hydroxypropyl cellulose.

7. The solid oral composition according to claim 4, **characterized in that** said lubricant is selected from the group comprising sodium stearyl fumarate, sodium lauryl sulphate, magnesium stearate, polyethylene glycol, metal stearates, hydrogenated castor oil and the like, and the mixtures thereof, preferably it is sodium stearyl fumarate.

8. The solid oral composition according to claim 4, **characterized in that** said glidant is selected from the group comprising stearic acid, colloidal silicon dioxide, talk, aluminium silicate and the like, and the mixtures thereof, preferably it is stearic acid.

9. The solid oral composition according to any of the preceding claims, **characterized in that** the amount of sodium stearyl fumarate is from 0.1 to 10%, preferably from 0.25 to 2% of the total weight of the tablet.

10. The solid oral composition according to any of the preceding claims, **characterized in that** the viscosity of low substituted hydroxypropyl cellulose is between 6.0-10.0 mPa.s.

11. The solid oral composition according to any of the preceding claims, **characterized in that** the amount of low substituted hydroxypropyl cellulose is from 1 to 50%, preferably from 6 to 25% of the total weight of the tablet.
